# EUROPEAN PATENT SPECIFICATION

(11) **EP 4 143 296 B1**
(45) Date of publication and mention of the grant of the patent: **14.02.2024**
(21) Application number: 21714987.1
(22) Date of filing: 25.03.2021
(51) Int. Cl.: C12M 3/06, B01L 3/00, G01N 33/483, G01N 33/50, B01L 9/00

(54) **ALL-IN-ONE MICROCHAMBER FOR 3D MUSCULAR TISSUES**
EINTEILIGE MIKROKAMMER FÜR 3D-MUSKELGEWEBE
MICROCHAMBRE TOUT-EN-UN POUR TISSUS MUSCULAIRES 3D

(30) Priority: 28.04.2020 NL 2025441
(43) Date of publication of application: 08.03.2023
(73) Proprietor: Biond Solutions B.V., 2628 CD Delft (NL)
(72) Inventor: GAIO, Nikolas, 2628 CD DELFT (NL); QUIROS SOLANO, William Fausto, 2628 CD DELFT (NL); OTHMAN, Amr Abdelhameed Mohamed, 2628 CD DELFT (NL); SILVESTRI, Cinzia, 2628 CD DELFT (NL)
(74) Representative: Hoyng Rokh Monegier B.V.
(86) International application number: PCT/NL2021/050201
(87) International publication number: WO 2021/221495

(56) References cited:
- WO-A1-2017/218581
- US-A1- 2014 038 225
- US-A1- 2019 168 218

## Description

### FIELD OF THE INVENTION

The present invention is in the field of an all-in-one microchamber for 3D muscular tissues, wherein at least one 3D microenvironment is present, a method of producing said device using silicon based technology, and a use of said device in various applications, typically a biological cell experiment, such as a cell or organ-on-a-chip experiment, and lab-on-a-chip experiment, and use of the device as a micro-reactor.

### BACKGROUND OF THE INVENTION

A microfluidic device relates to a set of technologies with an aim to manipulate at least one small fluid (liquid or gas) volume within microsystems produced by human beings. In the device a cell culture or an individual cell or the like may be present. An experiment on said cell culture refers to the maintenance and growth of cells in a well-controlled environment. The environment may resemble naturally occurring circumstances. As such a cell can likewise be studied under application of at least one of numerous signals that might be present in its naturally occurring surrounding microenvironment.

A microfluidic cell culture may attempt to manipulate cells, such as by culturing, maintaining, and growing, and qualitatively and quantitatively experimenting and analyzing cells in microfluidic volumes. Such may relate to an attempt to understand a cell culture, such as a stem cell culture, non-dividing or slowly dividing cells, e.g. in terms of an interaction between cell culture parameters and the micro environmental conditions created by microfluidic devices. It is considered that dimensions of the microfluidics, such as chamber and channels, are well suited to the physical scale of the biological cells and other applications.

In general, it is considered that microfluidics provide a good degree control over e.g. cell culture conditions. Typically a movement of fluids in the microfluidics is considered to be laminar; a fluid volume is typically in the order of 10⁻⁶-10⁻¹² l, which volume may flow in 10-60 seconds; fluid flow may be controlled precisely in terms of volume and timing, such as by providing an in-chip valve; also precise chemical and physical control of the microenvironment is possible; a production of a multitude of individually controllable cell culture chambers on a single device is considered, albeit typical prior art technologies rely on manual procedures which are considered to be insufficiently controlled.

Some prior art documents recite microfluidic devices. WO2016/049363 A1, WO2016/049365 A1, WO2016/010861 A1, WO2016/004394 A1, and US15/2955534 A1 recite relatively simple organ-on-chip devices, which cannot include any complex sensing/stimulation elements; hence these devices are not unsuited for most applications. US2014/038225 is also prior art.

The present inventors have filed an international application, WO 2018/021906 A1, comprising basic concepts of such microfluidic devices.

Further developments have been aimed at certain aspects of microchambers with advanced functionality, but typically at one aspect at a time. For instance, supports for a gel matrix can be provided. Or a reaction chamber with advanced microfluidic channels providing flow paths is provided, aimed at trapping cells or the like. Or cantilevers for supporting cells. Or media for supporting cell maturation.

The present invention relates to a device and a method of producing a versatile and advanced device which overcomes one or more of the above or further disadvantages, without jeopardizing functionality and advantages.

### SUMMARY OF THE INVENTION

The present invention relates in a first aspect to a micro-fluidic device 100 comprising at least one first microchamber 10a having a bottom 11 and at least one wall 12, preferably wherein the first microchamber comprises an opening such that it is directly accessible from outside, in the microchamber at least one pillar 20 extending from the bottom 11 upwards for supporting of cell or tissue, i.e. pillars are resting on the bottom, preferably wherein the at least one pillar has a height of 1-2000 pm, and preferably wherein the at least one pillar has a width of 1-2000 µm, and at least one first channel 30 in fluidic contact with the at least one microchamber 10a embedded in the bottom 11, wherein the bottom 11 comprises a porous membrane 13 so as to embody a selective barrier providing fluidic contact between the at least one first channel 30 and the at least one microchamber 10a. The porous membranes allows to selectively isolate the microfluidic channel, allowing to inject cells inside the channel (without having them enter in the microchamber), and cover the four walls of the channel, creating a structure that resembles a blood vessel very close to the tissue in the microchamber. The pores in the membrane can be designed so that some cells do not pass through them, while nutrients do. Moreover, the pores can be designed to allow for angiogenesis and vascularization starting from the microfluidic channel.

The device according to claim 1 has further advantages of a higher throughput, being cheaper to produce, being more reliable and more versatile, providing a better handling of e.g. cells, and providing a much wider functionality.
First and second microchambers typically have a dimension (e.g. cross-section) in the order of 5-1000 pm, preferably 10-500 µm such as 100-300 µm. First and second microchannels typically have a dimension (e.g. cross-section) in the order of 50-1000 pm, preferably 100-700 µm such as 400-500 µm.

The present device comprises at least two distinct layers in which microfluidic and nano-/microscale elements and the like are provided. Two layers are typically made of a polymer, typically but not necessarily the same polymer for both layers; a first polymer layer 13a is provided on a substrate, typically silicon or a glass wafer, and is relatively thin; for the purpose of the invention the terms "substrate", "silicon", and "glass" are considered interchangeable; the top layer may be considered to relate to a membrane, which is considered to relate to a selective barrier; the top layer preferably is provided with a matrix of openings (or holes) 11a therein, the at least one hole allowing passage of e.g. fluids, gases, species, microparticles, ions, etc. which can be adapted for specific uses; the top layer has a thickness of 0.05-30 pm, preferably 0.1-25 pm, more preferably 0.2-20 pm, even more preferably 0.5-10 µm thin, such as 1-8 µm or 2-6 µm; in contact with the relative thin polymer layer 13a is a thicker polymer bottom layer 13b; the bottom layer may comprise at least one second micro-channel 31 and/or at least one second micro-chamber 10b at least partly embedded in the polymer bottom layer; the number, layout, sizes, and further characteristics of these microfluidics can be adapted for specific uses; the microfluidics may be embedded fully in the bottom layer 13b and/or may be embedded partly, such as in the case of a well; the polymer bottom layer is thicker than the top layer and preferably has a thickness of 50-2000 pm, hence is at least one order of magnitude thicker than the top layer, and typically 2-3 orders of magnitude thicker; the thickness is preferably 150-1000 pm, more preferably 200-500pm, even more preferably 250-400 µm; the device further comprises silicon based microfluidics in microfluidic contact with the top layer 13a of the polymer based microfluidics wherein the silicon based microfluidics are accessible and/or can be made accessible for use of the device; the substrate, e.g. silicon, based microfluidics comprise at least one first micro-channel 30 and/or at least one first micro-chamber 10a at least partly embedded (see above) in the silicon, and may comprise at least one input 16, wherein the input 16 is in microfluidic contact with the at least one second micro-channel 31 and/or at least one second micro-chamber 10b embedded in the polymer bottom layer, e.g. as functionally defined or required; the support or substrate 10 may relate to a typically used wafer in a silicon semiconductor process such as of Si or glass; wherever silicon is mentioned in this respect it may relate to any other suitable substrate; the polymer top layer 13a is for separating (fluidics in the) at least one of the first micro-channel 30 and/or at least one of the first micro-chamber 10a embedded in the substrate (silicon) from (fluidics in the) at least one of the second micro-channel 31 and/or at least one of the second micro-chamber 10b embedded in the polymer bottom layer preferably at least partly by the matrix of holes 11a therein; the microfluidics of the polymer and silicon are directly or indirectly in microfluidic contact with one and another. The present polymer is independently selected from biocompatible polymers, such as polysiloxanes, such as polydimethylsiloxane (PDMS), polyimides, polyurethane, styrene-ethylene-butylene-styrene (SEBS), polypropylene, polycarbonate, polyester, polypropylene, and butyl rubber, and from biodegradable polymers, such as Biorubber (poly(glycerol sabacate PGS), and poly(1,8-octanediol-co-citrate) (POC), and combinations thereof.

The term "fluidics" may relate to a gas, a liquid; and combinations thereof; a "microfluidic" is considered to relate to a fluid under boundary conditions of the device.

The set-up composed by the polymer layers, typically forming a polymer film, the at least one micro-channel, such as 2-10 micro-channels, the at least one micro-chamber, such as 2-10 microchambers, and first micro-chamber (also referred to as "macro-chamber") can be optically monitored off-line with a microscope and/or a camera e.g. placed on a backside/front-side of the device. In many of the examples only one macro-chamber 10a is present. The set-up can be monitored on-line by means of micro-electrode array and/or micro-fabricated sensors (such as flow/temperature/pH sensor) placed in the micro-environment and or in the macro-chambers. The set-up can be also altered/stimulated by means of liquid flow flowing through the micro-chamber/channels and the macro-chamber; likewise by gas flow flowing through the micro-chamber/channel and the macro-chamber; by pressure differences applied in the micro-chambers, in the micro-channels and on the backside and the front side of the polymer film; by electrical stimulation provided by means of microelectrode arrays; by optical stimulation provided with optical systems placed on the backside/front-side of the device; by chemical stimulation provided by means of liquid flow or liquid reservoir placed in the membrane; and other micro-fabricated actuators placed inside the micro-channel/chambers; and combinations thereof, hence the device is considered to be versatile.

In a second aspect the present invention relates to a method of applying a stimulus to at least one living organism or living part thereof, comprising providing at least one micro-fluidic device (100) according to the invention, providing the at least one living organism or living part thereof each individually comprising at least one cell, such as in microchamber 10a or in channel 30, providing at least one stimulus to the at least one living organism or living part thereof, and obtaining a test result.

The terms "at least one living organism or living part thereof" are considered to relate to actual organisms, parts thereof, such as cell lines, tissue, etc., biological material resembling living organisms or parts thereof, and any other similar form of biological material, such as identified throughout the application and claims.

Thereby the present invention provides a solution to one or more of the above-mentioned problems.

Advantages of the present invention are detailed throughout the description.

### DETAILED DESCRIPTION OF THE INVENTION

The present invention relates in a first aspect to a device according to claim 1.

In an exemplary embodiment the present device may further comprise at least one stimulator 40, such as an electrical stimulator 41, such as an electrode, a chemical stimulator, an optical stimulator, and a mechanical stimulator 42, such as a pump pneumatical pressure/force, and/or a tissue monitor, such as an electrode. With said stimulator an effect thereof can be monitored or studied on the living organism, or part thereof.

In an exemplary embodiment of the present device the bottom 11 may comprise at least one opening 11a, preferably an array of openings 11a, such as 1-100 openings 11a/µm².

In an exemplary embodiment of the present device the array comprises nxm openings, wherein n>10, and m>10.

In an exemplary embodiment of the present device a density of holes may be 0.001-250/100 µm².

In an exemplary embodiment of the present device an average hole area may be 0.05-500 µm².

In an exemplary embodiment of the present device the at least one pillar may preferably be provided near and/or on top of the at least one opening.

In an exemplary embodiment of the present device the bottom 11 may comprise a polymer film 13, preferably wherein the polymer film comprises a 0.05-100 µm thin polymer top layer 13a the polymer top layer preferably having a matrix of holes 11a therein.

In an exemplary embodiment of the present device the bottom 11 may comprise a 50-5000 µm polymer bottom layer 13b in contact with the polymer top layer, and optionally comprising at least one second micro-channel 31 and/or at least one second micro-chamber10b at least partly embedded in the polymer bottom layer.

In an exemplary embodiment of the present device the at least one pillar is preferably provided near and/or on top of the at least one first or second micro-chamber.

In an exemplary embodiment of the present device wherein the at least one pillar is preferably provided near and/or on top of the at least one first or second micro-channel.

In an exemplary embodiment the at least one pillar is selectable to be of a rigid construction or of a flexible construction. The advantage of this aspect is that the functionality of the pillars can be increased (for example, by being used as 3D electrodes). Moreover, with pillars that can be both flexible and rigid, the forces experienced by the tissues anchored to the two pillars can be tuned.

In an exemplary embodiment of the present device the polymer may independently be selected from biocompatible polymers, such as poly siloxanes, such as polydimethylsiloxane PDMS, polyimides, polyurethane, butyl rubber, styrene-ethylene-butylene-styrene SEES, polypropylene, polycarbonate, polyester, polypropylene, and biodegradable polymers, such as Biorubber PGS and poly1,8-octanediol-co-citrate POC, and combinations thereof.

In an exemplary embodiment of the present device the thin polymer top layer 13a may comprise at at least one side thereof, at least one micro-feature, such as an indentation, a groove, a topographical structure, preferably at least one oriented microgroove, preferably an array of x*y oriented microgrooves, wherein a density of microgrooves is 1-25/100 µm².

In an exemplary embodiment of the present device an average groove area may be 0.1-10⁶ µm².

In an exemplary embodiment of the present device the at least one micro-feature may be aligned with respect to the device.

In an exemplary embodiment of the present device the polymer layers 13a,13b are provided with at least one access, the at least one access providing access to at least one of a metal pad, an IC, a sensor, such as an optical sensor, and a heater.

In an exemplary embodiment of the present device a rigid substrate may form the microchamber wall 12, such as a silicon substrate.

In an exemplary embodiment of the present device the least one first microchamber 10a may have a shape resembling a biological tissue or organism to be received.

In an exemplary embodiment the present device may comprise at least one electrode 41 in the bottom.

In an exemplary embodiment of the present device the electrode is preferably provided in the bottom top layer 11a.

In an exemplary embodiment of the present device the electrode is provided in microchannel 30.

In an exemplary embodiment of the present device the electrode is provided on layer 13a.

In an exemplary embodiment of the present device an electrode at one end may be in electrical contact with at least one pillar 20.

In an exemplary embodiment of the present device the electrode may comprise a contact 41a at another end thereof, such as a pad, wherein optionally the electrode is incorporated in an insulating material 41b, and wherein optionally the contact 41a is electrically separated from the wall 12 by a further insulating material 41c. It is possible to contact the electrode with the contact 41a through an intermediate metal line, which may be straight or meandering or which may follow any other suitable path.

In an exemplary embodiment of the present device the bottom may comprise a metal layer 13c, preferably in between a top layer 13a and bottom layer 13b, wherein the metal layer is preferably patterned, and wherein the metal layer is adapted to detect deformation of the at least one pillar, such as for strain gauges that measure the bending of the pillars to detect the contraction of the cell bundle attached to the pillars.

In an exemplary embodiment of the present device the at least one pillar 20 may have a cross-section selected from square, rectangular, oval, elliptic, circular, triangular, multigonal, and combinations thereof.

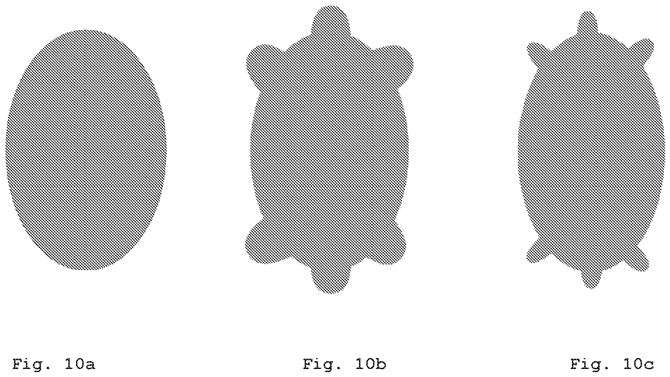

The shape of the first microchamber can be tailored to reduce the stress inflicted on the electrodes and the intermediate metal lines. A possible option is for instance to apply the cross-sectional shape shown in figure 10b and 10c, which depicts an essentially elliptical shape provided with three protrusions on the opposite heads of the virtual relatively long axis of the ellipse.

In an exemplary embodiment of the present device a cross-section may be substantially constant from the bottom upwards, or wherein the cross-section gradually may increase in area from the bottom upwards.

In an exemplary embodiment of the present device at least one pillar may be provided with an optical guider 23 at a top thereof, such as a pointer, such as a triangular pointer, wherein optical guiders of opposite pillars may point in the same direction, or in an opposite direction, and combinations thereof.

In an exemplary embodiment of the present device at least one pillar may be hollow, preferably wherein the hollow part of the pillar is in microfluidic connection with channel 30, or with channel 31, or with microchamber 10a or 10b.

In an exemplary embodiment the present device may further comprise embedded in the device at least one of a pump, or adapted to receive fluid from a pump, a valve, a strain gauge, an actuator, a heater, a cooler, a flow sensor, a temperature sensor, a pH sensor, an IC-circuit, an amplifier, an actuator, a hot plate, a micro-electrode array, an ion sensor, a pressure regulator, further microfluidic elements, at least one of a microchip, an integrated sensor, and an output 18, preferably embedded in the bottom 11, such as in a rigid part thereof.

In an exemplary embodiment of the present device a wet/humid section and a dry section of the device may be physically separated, wherein the dry section comprises electronics.

In an exemplary embodiment of the present device the bottom may comprise a polymer film which is stretchable having a tensile strength of > 1 [MPa] (ISO 527) and/or flexible with a Young's modulus of <3 [GPa] (ISO 527).

In an exemplary embodiment of the present device the polymer film may be rigid having a Young's modulus of >10 [GPa] (ISO 527).

In an exemplary embodiment the present device may further comprise a support 60, such as a plate, wherein the micro-fluidic device is preferably detachably attached to said support.

In an exemplary embodiment the present device may comprise at least one living organism or living part thereof, wherein the at least one living organism is selected from undifferentiated cells, differentiated cells, mature cells, stem cells, such as adherent or suspension primary cells, transfected or non-transfected cell lines, adult, embryonic or induced pluripotent stem cells, tissues, tissues inserts, and 3D microtissues, such as muscles and cardiac micro tissues, 3D cultures, such as spheroids and organoids, and combinations thereof.

In an exemplary embodiment of the present method the stimulus may be a chemical stimulus, a mechanical stimulus, an electrical stimulus, or an optical stimulus, such as for toxicity testing of drugs and xenobiotics, for efficacy testing, for modeling barrier tissues in vitro, for integrity assessment and drug transport assays, for Drug metabolism studies, for drug pharmacokinetic and toxicokinetic studies, for metabolizing organ and targeting pharmacological organs, for disease modelling, for disease diagnosis, for studying a disease mechanism, for prognosis, for personalized and precise medicine, for doping, for microgravity studies, for drug interaction, for cell maturation, and for cell differentiation.

In an exemplary embodiment of the present method the method is in vitro.

In an exemplary embodiment of the present method the at least one living organism may be selected from undifferentiated cells, differentiated cells, mature cells, stem cells, such as adherent or suspension primary cells, transfected and non-transfected cell lines, adult cells, embryonic and induced pluripotent stem cells, tissues, tissues inserts, clustered cells, printed cells, an organoid, tissue biopsy, tumor tissue, resected tissue material, an organ explant, an embryonic body, and 3D microtissues, such as muscles and cardiac micro tissues, 3D cultures, such as spheroids and organoids, and combinations thereof.

The invention is further detailed by the accompanying figures and examples, which are exemplary and explanatory of nature and are not limiting the scope of the invention. To the person skilled in the art it may be clear that many variants, being obvious or not, may be conceivable falling within the scope of protection, defined by the present claims.

### FIGURES

Figures 1a-d, 2a-c, 3a-f, 4a-d, 5a-e, 6a-d, 7-8, and 9a-d show details of an exemplary embodiment of the present device and method.
Figure 10 shows possible shapes of the microchamber of the device.

### DETAILED DESCRIPTION OF THE FIGURES

In the figures the numbers below depict the features that are mentioned thereafter:
- 100: Micro-fluidic Device
- 10a: first microchamber
- 10b: second microchamber
- 11: bottom of first microchamber
- 11a: opening
- 12: wall of first microchamber
- 13: polymer film
- 13a: thin polymer top layer
- 13b: polymer bottom layer
- 13c: metal bottom layer
- 18: output
- 20: pillar
- 23: optical guider
- 30: first microchannel
- 31: second microchannel
- 40: stimulator
- 41: electrical stimulator
- 41a: electrode contact
- 41b: electrode insulator material
- 41c: electrode/wall insulator
- 42: mechanical stimulator
- 43: silicon
- 50: tissue (e.g. muscle bundle)
- 60: support

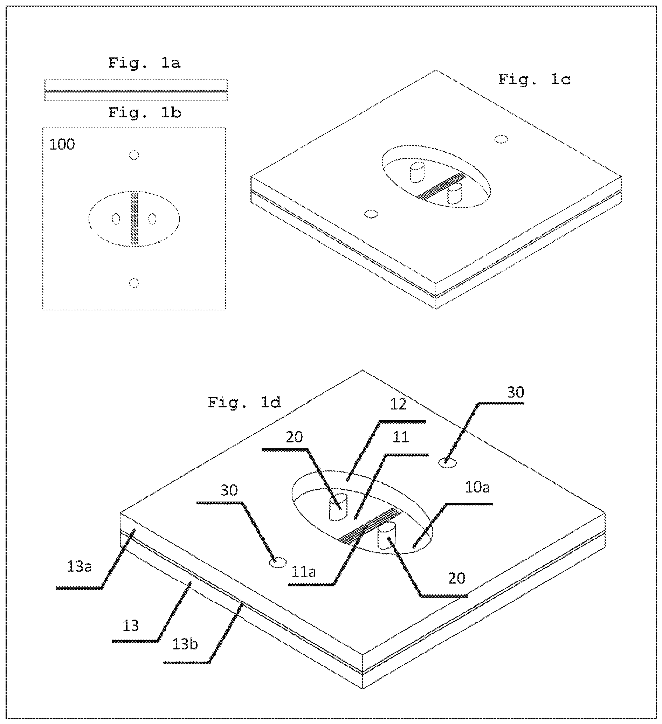

Figures 1a-d show details of an exemplary embodiment of one device that includes two pillars, one channel accessible through inlet and an outlet and through hole matrix in the channel. Figure 1a: side view. Figure 1b: top view.

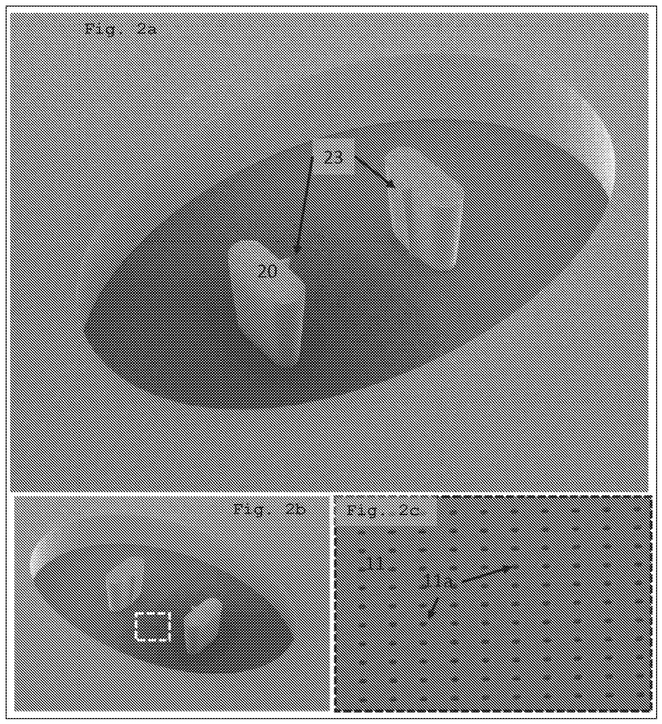

Figs. 2a-c show details of an exemplary embodiment of one device with oval well, equipped with two pillars and a microfluidic channel. Figure 2a shows details of an exemplary embodiment of one device with pointer that can be used as optical guiders to identify the displacement of the pillars caused by the contraction of the muscle bundle. Figure 2c shows a close up of an array of openings present in the device.

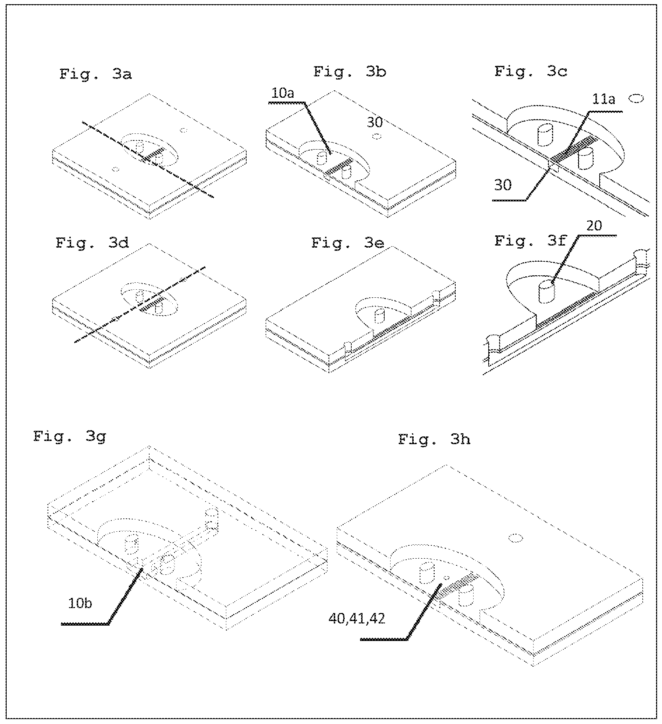

Figures 3a-f shows details of the microfluidic channel inside the chip. Figs. 3b-c are cross-sections of fig. 3a, and figs. 3e-f are cross-sections of fig. 3d.

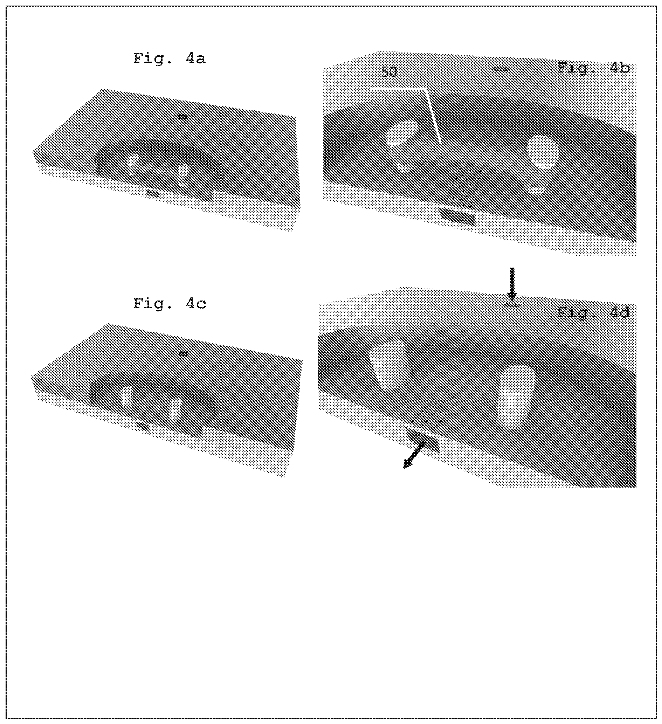

Figs. 4a-d show images of details of the present device with (Figure 4a,b) and without (Figure 4c,d) a muscle bundle supported by the two pillars present in the device. The arrow indicates a flow path.

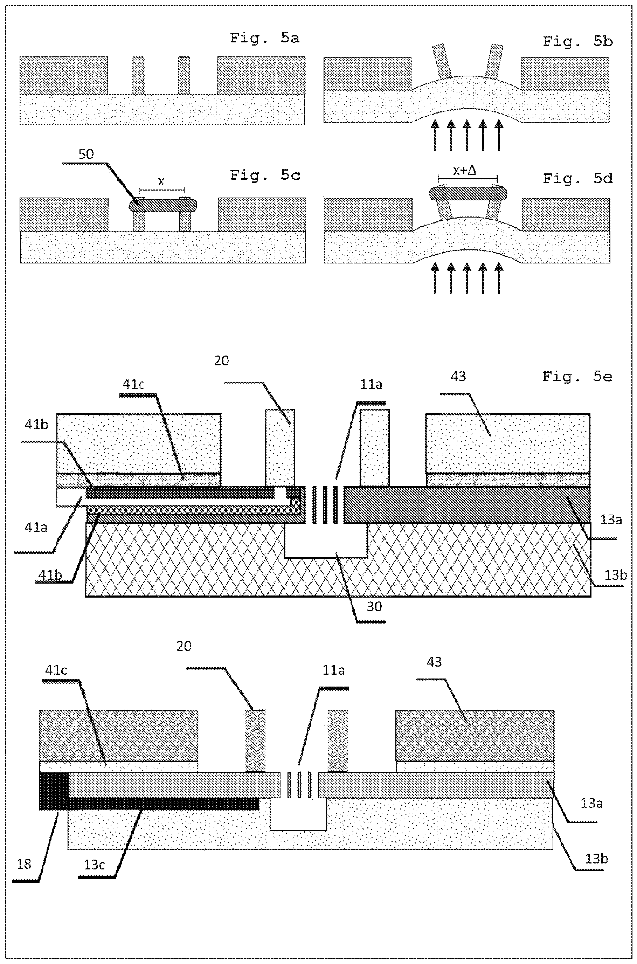

Figs. 5a-e show cross-sectional details of the present invention. Figures 5a-e show details of an exemplary embodiment of one device when it is in relaxed state with and without muscle bundle, when the polymer layers are stretched by applying a difference of pressure between the microchamber and the back of the thick polymer layer. Figure 5e shows details of an exemplary embodiment of one device with a pillars electrically accessible via contact pads, to stimulate and monitor the tissue and cells inserted in the chip. The figure reflects a micro-fluidic device further comprising at least one electrode (41) in the bottom, wherein the electrode is preferably provided in the bottom top layer (11a), wherein an electrode at one end is in electrical contact with at least one pillar (20), and wherein the electrode comprises a contact (41a) at another end thereof, such as a pad, wherein optionally the electrode is incorporated in an insulating material (41b), and wherein optionally the contact (41a) is electrically separated from the wall (12) by a further insulating material (41c).) In fig. 5e contact pad 41a, and pillar 20 being electrically accessible via contact pad 41a, are shown.

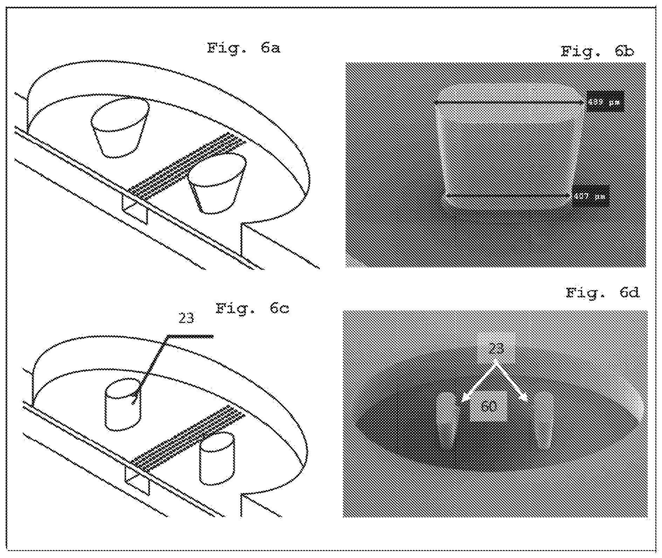

Figure 6a-b show details of an exemplary embodiment of one device wherein the cross-section of the pillars gradually increases in area from the bottom upwards. Figure 6c-d shows details of an exemplary embodiment of one device with pointer that can be used as optical guiders to identify the displacement of the pillars caused by the contraction of the muscle bundle.

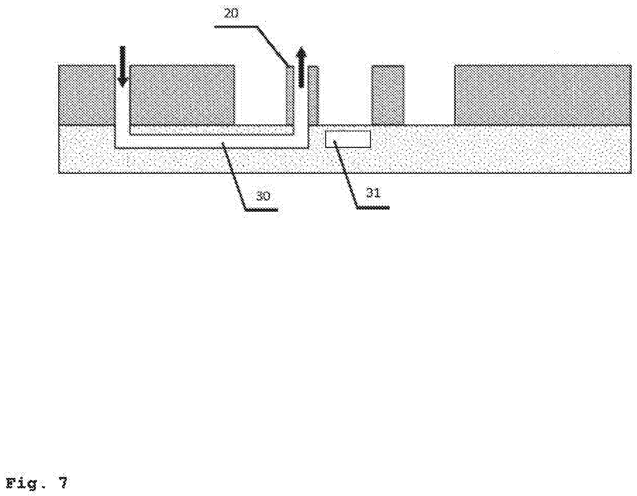

Fig. 7 shows a further cross-section of an exemplary embodiment of one device wherein at least one pillar (20) is hollow, preferably wherein the hollow part of the pillar (20) is in microfluidic connection with first channel (30), or with second channel (31), or with first or second microchamber (10a, 10b). The arrow indicates a flow path.

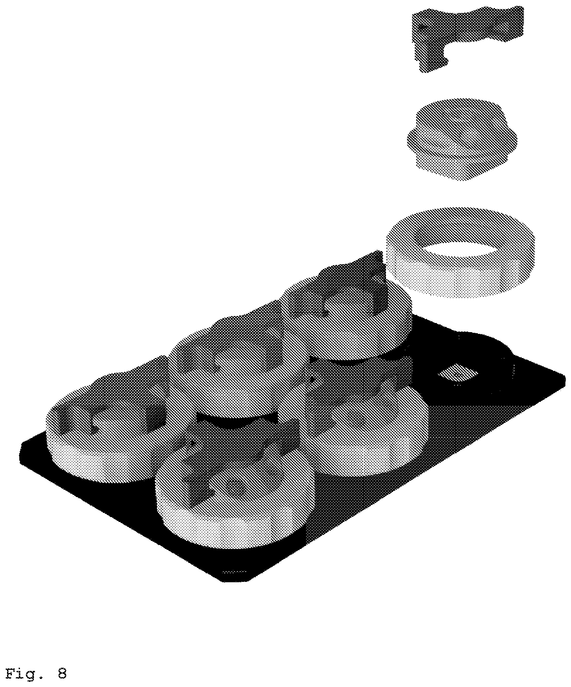

Fig. 8 shows a device comprising a support (60), such as a plate, wherein the micro-fluidic device is preferably detachably attached to said support (60).

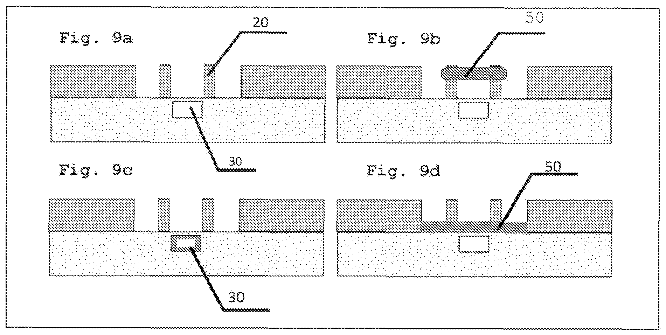

Fig. 9 shows a further cross-section of an exemplary embodiment of one device without (a) and with (b, c, d) cells cultured inside the device. The pillars (20) can be employed to support a muscle bundle (50) (Fig. 9b). Cells can be also cultured inside the microfluidic channel (Fig. 9c) or on the bottom of the macro chamber (Fig. 9d). Cell cultures shown in Figure 9b, c, d can be combined.

Fig. 10 depict several possible shapes of the microchamber of the device of the invention.

### EXAMPLES/EXPERIMENTS

The invention although described in detailed explanatory context may be best understood in conjunction with the accompanying examples and figures.

## Claims

1. Micro-fluidic Device (100) comprising
at least one first microchamber (10a) having a bottom (11) and at least one wall (12), preferably wherein the first microchamber (10a) comprises an opening such that it is directly accessible from outside,
in the first microchamber (10a) at least one pillar (20) extending from the bottom (11) upwards for supporting of cell or tissue, preferably wherein the at least one pillar has a height of 1-5000 pm, and preferably wherein the at least one pillar has a width of 1-2000 pm, and
at least one first channel (30) in fluidic contact with the at least one microchamber (10a) embedded in the bottom (11), **characterized in that** the bottom (11) comprises a porous membrane (13) so as to embody a selective barrier providing fluidic contact between the at least one first channel (30) and the at least one microchamber (10a).

2. Micro-fluidic device according to claim 1, further comprising at least one stimulator (40), such as an electrical stimulator (41), such as an electrode, a chemical stimulator, an optical stimulator, and a mechanical stimulator (42), such as a pump, and/or a tissue monitor, such as an electrode.

3. Micro-fluidic device according to any of claims 1-2,
wherein the porous membrane (13) comprises at least one opening (hole) (11a), preferably an array of openings (11a), such as 1-100 openings/µm², and/or
wherein the array comprises nxm openings, wherein n>10, and m>10,
wherein a density of openings is 0.001-250/100 µm², and/or
wherein an average openings area is 0.05-500 µm², and/or
wherein the at least one pillar is preferably provided near and/or on top of the at least one opening.

4. Micro-fluidic device according to any of claims 1-3, wherein the porous membrane (13) comprises a 0.05-100 µm thin polymer top layer (13a), the polymer top layer preferably having a matrix of holes (11a) therein,
a 50-5000 µm thin polymer bottom layer (13b) in contact with the polymer top layer(13a), and optionally comprising at least one second micro-channel (31) and/or at least one second micro-chamber(10b) at least partly embedded in the polymer bottom layer (13b),
wherein the at least one pillar (20) is preferably provided near and/or on top of the at least one first or second micro-chamber (10a,10b) wherein the at least one pillar (20) is preferably provided near and/or on top of the at least one first or second micro-channel (30,31), and/or
wherein the polymer of the polymer film (13) is independently selected from glass, silicon oxide, silicon nitride or biocompatible polymers, such as poly siloxanes, such as polydimethylsiloxane (PDMS), polyimides, polyurethane, butyl rubber, styrene-ethylene-butylene-styrene (SEBS), polypropylene, polycarbonate, polyester, polypropylene, and biodegradable polymers, such as Biorubber (poly(glycerol sebacate PGS) and poly(1,8-octanediol-co-citrate) (POC), and combinations thereof, and/or
wherein the thin polymer top layer (13a) comprises at at least one side thereof, at least one micro-feature, such as an indentation, a groove, a topographical structure, preferably at least one oriented microgroove, preferably an array of x*y oriented microgrooves, wherein a density of microgrooves is 1-25/100 µm², and/or
wherein an average groove area is 0.1-10⁶ µm², and/or
wherein the at least one micro-feature is aligned with respect to the device, and/or
wherein the polymer layers (13a,13b) are provided with at least one access, the at least one access providing access to at least one of a metal pad, an IC, a sensor, such as an optical sensor, and a heater, and/or
a rigid substrate forming the microchamber wall (12), such as a silicon substrate, and/or
wherein the least one first microchamber (10a) has a shape resembling a biological tissue or organism to be received.

5. Micro-fluidic device according to any of claims 1-4, comprising at least one electrode (41) in the bottom (11), wherein the electrode (41) is preferably provided in the bottom top layer (11a),
wherein an electrode at one end is in electrical contact with at least one pillar (20), and wherein the electrode comprises a contact (41a) at another end thereof, such as a pad, wherein optionally the electrode (41) is incorporated in an insulating material (41b),
and wherein optionally the contact (41a) is electrically separated from the wall (12) by a further insulating material (41c).

6. Micro-fluidic device according to any of claims 1-5, wherein the porous membrane (13) and/or the at least one pillar (2) is one selected of a rigid construction and a flexible construction.

7. Micro-fluidic device according to any of claims 1-6, wherein the bottom comprises a metal layer (13c), preferably in between a top layer (13a) and bottom layer (13b), wherein the metal layer is preferably patterned, and wherein the metal layer is adapted to detect deformation of the at least one pillar.

8. Micro-fluidic device according to any of claims 1-7,
wherein the at least one pillar (20) has a cross-section selected from square, rectangular, oval, elliptic, circular, triangular, multigonal, and combinations thereof, and/or
wherein a cross-section is substantially constant from the bottom (11) upwards, or wherein the cross-section gradually increases in area from the bottom (11) upwards, and/or
wherein at least one pillar is provided with an optical guider (23) at a top thereof, such as a pointer, such as a triangular pointer, optionally wherein optical guiders of opposite pillars may point in the same direction, or in an opposite direction, and combinations thereof, and/or
wherein at least one pillar (20) is hollow, preferably wherein the hollow part of the pillar (20) is in microfluidic connection with first channel (30), or with second channel (31), or with first or second microchamber (10a, 10b).

9. Micro-fluidic device according to any of claims 1-8, further comprising embedded in the device at least one of a pump, or wherein the device is adapted to receive fluid from a pump, a valve, a strain gauge, an actuator, a heater, a cooler, a flow sensor, a temperature sensor, a pH sensor, an IC-circuit, an amplifier, an actuator, a hot plate, a micro-electrode array, an ion sensor, a pressure regulator, further microfluidic elements, at least one of a microchip, an integrated sensor, and an output (18), preferably embedded in the bottom (11), such as in a rigid part thereof, and/or wherein a wet/humid section and a dry section of the device are physically separated, wherein the dry section comprises electronics.

10. Micro-fluidic device according to any of claims 1-9, wherein the bottom (11) comprise a polymer film (13) which is stretchable having a tensile strength of > 1 [MPa] (ISO 527), and/or flexible with a Young's modulus of <3 [GPa] (ISO 527), or wherein the polymer film is rigid having a Young's modulus of >10 [GPa] (ISO 527).

11. Micro-fluidic device according to any of claims 1-10, further comprising a support (60), such as a plate, wherein the micro-fluidic device is preferably detachably attached to said support (60).

12. Micro-fluidic device according to any of claims 1-11, comprising at least one living organism or living part thereof, wherein the at least one living organism is selected from undifferentiated cells, differentiated cells, mature cells, stem cells, such as adherent or suspension primary cells, endothelial cells, transfected or non-transfected cell lines, adult, embryonic or induced pluripotent stem cells, tissues, tissues inserts, and 3D microtissues, such as muscles and cardiac micro tissues, 3D cultures, such as spheroids and organoids, and combinations thereof.

13. Method using the device according to any of claims 1-11, comprising seeding the first microchamber (10a) with primary or induced pluripotent stem cell skeletal or cardiac muscle cells with or without myoblasts/ fibroblasts/ endothelial cells to enable growth of a muscle bundle anchored to the pillars; and seeding the channel (30) with primary, transfected or induced pluripotent stem cell derived endothelial cells to create a 3D perfusable culture and/or to vascularize the bundle through the porous membrane.

14. Method of applying a stimulus to at least one living organism or living part thereof, comprising
providing at least one micro-fluidic device (100) according to any of claims 1-12,
providing the at least one living organism or living part thereof each individually comprising at least one cell,
providing at least one stimulus, and
obtaining a test result.

15. Method according to claim 14, wherein the stimulus is a chemical stimulus, a mechanical stimulus, an electrical stimulus, or an optical stimulus, such as for toxicity testing of drugs and xenobiotics, for efficacy testing, for modeling barrier tissues in vitro, for integrity assessment and drug transport assays, for Drug metabolism studies, for drug pharmacokinetic and toxicokinetic studies, for metabolizing organ and targeting pharmacological organs, for disease modelling, for disease diagnosis, for studying a disease mechanism, for prognosis, for personalized and precise medicine, for doping, for astronauts, for drug interaction, for cell maturation, and for cell differentiation.

16. Method according to any of claims 14-15, wherein the at least one living organism or living part thereof is selected from undifferentiated cells, differentiated cells, mature cells, stem cells, such as adherent or suspension primary cells, endothelial cells, transfected and non-transfected cell lines, adult cells, embryonic and induced pluripotent stem cells, tissues, tissues inserts, clustered cells, printed cells, an organoid, tissue biopsy, tumor tissue, resected tissue material, an organ explant, an embryonic body, and 3D microtissues, such as muscles and cardiac micro tissues, 3D cultures, such as spheroids and organoids, and combinations thereof.

## Patentansprüche

1. Mikrofluidische Vorrichtung (100), umfassend
mindestens eine erste Mikrokammer (10a), die einen Boden (11) und mindestens eine Wand (12) aufweist, vorzugsweise wobei die erste Mikrokammer (10a) eine Öffnung derart umfasst, dass sie von außen direkt zugänglich ist,
in der ersten Mikrokammer (10a) mindestens eine Säule (20), die sich von dem Boden (11) zum Tragen einer Zelle oder von Gewebe nach oben erstreckt, vorzugsweise wobei die mindestens eine Säule eine Höhe von 1-5000 µm aufweist, und vorzugsweise wobei die mindestens eine Säule eine Breite von 1-2000 µm aufweist, und
mindestens einen ersten Kanal (30) in fluidischem Kontakt mit der mindestens einen Mikrokammer (10a), die in den Boden (11) eingebettet ist, **dadurch gekennzeichnet, dass** der Boden (11) eine poröse Membran (13) umfasst, um eine selektive Barriere zu verkörpern, die einen fluidischen Kontakt zwischen dem mindestens einen ersten Kanal (30) und der mindestens einen Mikrokammer (10a) bereitstellt.

2. Mikrofluidische Vorrichtung nach Anspruch 1, ferner umfassend mindestens einen Stimulator (40), wie einen elektrischen Stimulator (41), wie eine Elektrode, einen chemischen Stimulator, einen optischen Stimulator und einen mechanischen Stimulator (42), wie eine Pumpe und/oder einen Gewebemonitor, wie eine Elektrode.

3. Mikrofluidische Vorrichtung nach einem der Ansprüche 1 bis 2,
wobei die poröse Membran (13) mindestens eine Öffnung (Loch) (11a), vorzugsweise eine Anordnung von Öffnungen (11a), wie 1-100 Öffnungen / µm² umfasst, und/oder
wobei die Anordnung nxm Öffnungen umfasst, wobei n>10, und m>10,
wobei ein Dichte von Öffnungen 0,001-250 / 100 µm² beträgt, und/oder
wobei eine durchschnittliche Öffnungsfläche 0,05-500 µm² beträgt, und/oder
wobei die mindestens eine Säule vorzugsweise nahe und/oder oben auf der mindestens einen Öffnung bereitgestellt ist.

4. Mikrofluidische Vorrichtung nach einem der Ansprüche 1 bis 3, wobei die poröse Membran (13) eine dünne Polymeroberschicht (13a) von 0,05-100 µm umfasst, wobei die Polymeroberschicht vorzugsweise eine Matrix von Löchern (11a) darin aufweist,
eine dünne Polymerbodenschicht (13b) von 50-5000 µm in Kontakt mit der Polymeroberschicht (13a) und optional umfassend mindestens einen zweiten Mikrokanal (31) und/oder mindestens eine zweite Mikrokammer (10b), die mindestens teilweise in die Polymerbodenschicht (13b) eingebettet ist,
wobei die mindestens eine Säule (20) vorzugsweise nahe und/oder oben auf der mindestens einen ersten oder zweiten Mikrokammer (10a, 10b) bereitgestellt ist, wobei die mindestens eine Säule (20) vorzugsweise nahe und/oder oben auf dem mindestens einen ersten oder zweiten Mikrokanal (30, 31) bereitgestellt ist, und/oder
wobei das Polymer des Polymerfilms (13) unabhängig aus Glas, Siliziumoxid, Siliziumnitrid oder biokompatiblen Polymeren ausgewählt ist, wie Polysiloxanen, wie Polydimethylsiloxan (PDMS), Polyimiden, Polyurethan, Butylkautschuk, Styrol-Ethylen-Butylen-Styrol (SEBS), Polypropylen, Polycarbonat, Polyester, Polypropylen und biologisch abbaubaren Polymeren, wie Biokautschuk (Poly(glycerinsebacat PGS) und Poly(1,8-octandiol-co-citrat) (POC) und Kombinationen davon, und/oder
wobei die dünne Polymeroberschicht (13a) an mindestens einer Seite davon, mindestens ein Mikromerkmal, wie eine Vertiefung, eine Nut, eine topographische Struktur, vorzugsweise mindestens eine ausgerichtete Mikronut, vorzugsweise eine Anordnung von x*y ausgerichteten Mikronuten umfasst, wobei eine Dichte von Mikronuten 1-25/100 µm² beträgt, und/oder
wobei eine durchschnittliche Nutfläche 0,1-10⁶ µm² beträgt, und/oder wobei das mindestens eine Mikromerkmal in Bezug auf die Vorrichtung ausgerichtet ist, und/oder
wobei die Polymerschichten (13a, 13b) mit mindestens einem Zugang versehen sind, wobei der mindestens eine Zugang zu mindestens einem von einer Metallplatte, einer IC, einem Sensor, wie einem optischen Sensor, und einer Heizung Zugang bereitstellt, und/oder
ein starres Substrat, das die Mikrokammerwand (12) ausbildet, wie ein Siliziumsubstrat, und/oder
wobei die mindestens eine erste Mikrokammer (10a) eine Form aufweist, die einem aufzunehmenden biologischen Gewebe oder Organismus ähnelt.

5. Mikrofluidische Vorrichtung nach einem der Ansprüche 1 bis 4, umfassend mindestens eine Elektrode (41) in dem Boden (11), wobei die Elektrode (41) vorzugsweise in der unteren Oberschicht (11a) bereitgestellt ist,
wobei eine Elektrode an einem Ende mit mindestens einer Säule (20) in elektrischem Kontakt steht, und wobei die Elektrode einen Kontakt (41a) an einem anderen Ende davon, wie einer Platte, umfasst, wobei optional die Elektrode (41) in ein Isoliermaterial (41b) integriert ist,
und wobei optional der Kontakt (41a) von der Wand (12) durch ein weiteres Isoliermaterial (41c) elektrisch getrennt ist.

6. Mikrofluidische Vorrichtung nach einem der Ansprüche 1 bis 5, wobei die poröse Membran (13) und/oder die mindestens eine Säule (2) eine ist, die aus einer starren Konstruktion und einer flexiblen Konstruktion ausgewählt ist.

7. Mikrofluidische Vorrichtung nach einem der Ansprüche 1 bis 6, wobei der Boden eine Metallschicht (13c) umfasst, vorzugsweise zwischen einer Oberschicht (13a) und einer Bodenschicht (13b), wobei die Metallschicht vorzugsweise strukturiert ist, und wobei die Metallschicht angepasst ist, um eine Verformung der mindestens einen Säule zu detektieren.

8. Mikrofluidische Vorrichtung nach einem der Ansprüche 1 bis 7,
wobei die mindestens eine Säule (20) einen Querschnitt aufweist, der aus quadratisch, rechteckig, oval, elliptisch, kreisförmig, dreieckig, multigonal und Kombinationen davon ausgewählt ist, und/oder
wobei ein Querschnitt von dem Boden (11) nach oben im Wesentlichen konstant ist oder wobei der Querschnitt von dem Boden (11) nach oben allmählich in der Fläche zunimmt, und/oder
wobei mindestens eine Säule mit einer optischen Führung (23) an einer Oberseite davon versehen ist, wie einem Zeiger, wie einem dreieckigen Zeiger, optional wobei optische Führungen von entgegengesetzten Säulen in die gleiche Richtung oder in eine entgegengesetzte Richtung und Kombinationen davon zeigen können, und/oder
wobei mindestens eine Säule (20) hohl ist, vorzugsweise wobei der hohle Teil der Säule (20) in mikrofluidischer Verbindung mit dem ersten Kanal (30) oder mit dem zweiten Kanal (31) oder mit der ersten oder zweiten Mikrokammer (10a, 10b) steht.

9. Mikrofluidische Vorrichtung nach einem der Ansprüche 1 bis 8, ferner umfassend eingebettet in die Vorrichtung mindestens eines von einer Pumpe, oder wobei die Vorrichtung angepasst ist, um Fluid von einer Pumpe aufzunehmen, ein Ventil, ein Dehnungsmesser, ein Antriebselement, einen Heizer, einen Kühler, einen Durchflusssensor, einen Temperatursensor, einen pH-Wert-Sensor, einen IC-Schaltkreis, einen Verstärker, ein Antriebselement, eine Heizplatte, eine Mikroelektrodenanordnung, einen Ionensensor, einen Druckregler, weitere mikrofluidische Elemente, mindestens eines von einem Mikrochip, einem integrierten Sensor und einem Ausgang (18), der vorzugsweise in den Boden (11) eingebettet ist, wie in einem starren Teil davon, und/oder wobei ein nass/feuchter Abschnitt und ein trockener Abschnitt physikalisch getrennt sind, wobei der trockene Abschnitt Elektronik umfasst.

10. Mikrofluidische Vorrichtung nach einem der Ansprüche 1 bis 9, wobei der Boden (11) einen Polymerfilm (13) umfasst, der dehnbar ist und eine Zugfestigkeit von >1 [MPa] (ISO 527) aufweist und/oder flexibel mit einem Elastizitätsmodul <3 [GPa] (ISO 527) ist, oder wobei der Polymerfilm starr ist und einen Elastizitätsmodul von >10 [GPa] (ISO 527) aufweist.

11. Mikrofluidische Vorrichtung nach einem der Ansprüche 1 bis 10, ferner umfassend einen Träger (60), wie eine Tafel, wobei die mikrofluidische Vorrichtung vorzugsweise an dem Träger (60) lösbar befestigt ist.

12. Mikrofluidische Vorrichtung nach einem der Ansprüche 1 bis 11, umfassend mindestens einen lebenden Organismus oder einen lebenden Teil davon, wobei der mindestens eine lebende Organismus aus undifferenzierten Zellen, differenzierten Zellen, reifen Zellen, Stammzellen, wie adhärenten oder suspendierten Primärzellen, Endothelzellen, transfizierten oder nicht transfizierten Zelllinien, adulten, embryonalen oder induzierten pluripotenten Stammzellen, Geweben, Gewebeeinsätzen und 3D-Mikrogeweben, wie Muskeln und Herzmikrogeweben, 3D-Kulturen, wie Sphäroiden und Organoiden und Kombinationen davon, ausgewählt ist.

13. Verfahren unter Verwendung der Vorrichtung nach einem der Ansprüche 1 bis 11, umfassend ein Animpfen der ersten Mikrokammer (10a) mit primären oder induzierten pluripotenten skelettartigen oder kardialen Stammzellenmuskelzellen mit oder ohne Myoblasten/Fibroblasten/Endothelzellen, um ein Wachstum eines Muskelbündels zu ermöglichen, das an den Säulen verankert ist; und Animpfen des Kanals (30) mit primären, transfizierten oder induzierten pluripotenten Stammzellen-abgeleiteten Endothelzellen, um eine 3D-perfundierbare Kultur zu erzeugen und/oder um das Bündel durch die poröse Membran zu vaskularisieren.

14. Verfahren zum Anlegen eines Stimulus an mindestens einen lebenden Organismus oder einen lebenden Teil davon, umfassend
Bereitstellen mindestens einer mikrofluidischen Vorrichtung (100) nach einem der Ansprüche 1 bis 12,
Bereitstellen des mindestens einen lebenden Organismus oder lebenden Teils davon, jeder einzeln umfassend mindestens eine Zelle,
Bereitstellen mindestens eines Stimulus und
Erhalten eines Testergebnisses.

15. Verfahren nach Anspruch 14, wobei der Stimulus ein chemischer Stimulus, ein mechanischer Stimulus, ein elektrischer Stimulus oder ein optischer Stimulus ist, wie für ein Toxizitätstesten von Arzneimitteln und Xenobiotika, für ein Wirksamkeitstesten, für ein Modellieren von Barrieregeweben in vitro, für Integritätsbewertung und Arzneimitteltransportassays, für Arzneimittelmetabolismusstudien, für pharmakokinetische und toxikoninetische Arzneimittelstudien, für ein Metabolisieren von Organen und ein Abzielen von pharmakologischen Organen, für ein Krankheitsmodellieren, für Krankheitsdiagnose, für ein Untersuchen eines Krankheitsmechanismus, für Prognose, für personalisierte und präzise Medizin, für ein Dotieren, für Astronauten, für Arzneimittelinteraktion, für Zellreifung und für Zelldifferenzierung.

16. Verfahren nach einem der Ansprüche 14 bis 15, wobei der mindestens eine lebende Organismus oder ein lebender Teil davon aus undifferenzierten Zellen, differenzierten Zellen, reifen Zellen, Stammzellen, wie adhärenten oder suspendierten Primärzellen, Endothelzellen, transfizierten oder nicht transfizierten Zelllinien, adulten, embryonalen oder induzierten pluripotenten Stammzellen, Geweben, Gewebeeinsätzen, Clusterzellen, gedruckten Zellen, einem Organoid, Gewebebiopsie, Tumorgewebe, Resektatmaterial, einem Organexplantat, einem Embryonalkörper und 3D-Mikrogeweben, wie Muskeln und Herzmikrogeweben, 3D-Kulturen, wie Sphäroiden und Organoiden und Kombinationen davon, ausgewählt ist.

## Revendications

1. Dispositif microfluidique (100) comprenant
au moins une première microchambre (10a) ayant un fond (11) et au moins une paroi (12), de préférence dans lequel la première microchambre (10a) comprend une ouverture de telle sorte qu'elle est directement accessible depuis l'extérieur,
dans la première microchambre (10a) au moins un pilier (20) s'étendant depuis le fond (11) vers le haut pour supporter une cellule ou un tissu, de préférence dans lequel l'au moins un pilier a une hauteur de 1 à 5000 µm, et de préférence dans lequel l'au moins un pilier a une largeur de 1 à 2000 µm, et
au moins un premier canal (30) en contact fluidique avec l'au moins une microchambre (10a) incorporée dans le fond (11), **caractérisé en ce que** le fond (11) comprend une membrane poreuse (13) de manière à réaliser une barrière sélective fournissant un contact fluidique entre l'au moins un premier canal (30) et l'au moins une microchambre (10a).

2. Dispositif microfluidique selon la revendication 1, comprenant en outre au moins un stimulateur (40), tel qu'un stimulateur électrique (41), tel qu'une électrode, un stimulateur chimique, un stimulateur optique et un stimulateur mécanique (42), tel qu'une pompe, et/ou un moniteur de tissu, tel qu'une électrode.

3. Dispositif microfluidique selon l'une quelconque des revendications 1 à 2,
dans lequel la membrane poreuse (13) comprend au moins une ouverture (trou) (11a), de préférence un réseau d'ouvertures (11a), tel que 1 à 100 ouvertures par µm², et/ou
dans lequel le réseau comprend n x m ouvertures, dans lequel n > 10, et m > 10,
dans lequel une densité d'ouvertures est de 0,001 à 250 par 100 µm², et/ou
dans lequel une zone d'ouvertures moyenne est de 0,05 à 500 µm², et/ou
dans lequel l'au moins un pilier est de préférence prévu près et/ou au-dessus de l'au moins une ouverture.

4. Dispositif microfluidique selon l'une quelconque des revendications 1 à 3, dans lequel la membrane poreuse (13) comprend une couche polymère supérieure mince de 0,05 à 100 µm (13a), la couche polymère supérieure ayant de préférence une matrice de trous (11a) dans celle-ci,
une couche polymère inférieure mince de 50 à 5000 µm (13b) en contact avec la couche polymère supérieure (13a), et comprenant éventuellement au moins un second microcanal (31) et/ou au moins une seconde microchambre(10b) au moins partiellement incorporée dans la couche polymère inférieure (13b),
dans lequel l'au moins un pilier (20) est de préférence prévu près et/ou au-dessus de l'au moins une première ou seconde microchambre (10a, 10b) dans lequel l'au moins un pilier (20) est de préférence prévu près et/ou sur le dessus de l'au moins un premier ou second microcanal (30, 31), et/ou
dans lequel le polymère du film polymère (13) est indépendamment choisi parmi du verre, de l'oxyde de silicium, du nitrure de silicium ou des polymères biocompatibles, tels que des poly-siloxanes, tels que du polydiméthylsiloxane (PDMS), des polyimides, du polyuréthane, du caoutchouc butyle, du styrène-éthylène-butylène-styrène (SEBS), du polypropylène, du polycarbonate, du polyester, du polypropylène, et des polymères biodégradables, tels que du biocaoutchouc (poly(glycérol sébacate PGS) et du poly(1,8-octanediol-co-citrate) (POC), et leurs combinaisons, et/ou
dans lequel la couche polymère supérieure mince (13a) comprend au moins un côté de celle-ci, au moins une micro-caractéristique, telle qu'une indentation, une rainure, une structure topographique, de préférence au moins une micro-rainure orientée, de préférence un réseau de x*y micro-rainures orientées, dans lequel une densité de micro-rainures est de 1 à 25 par 100 µm², et/ou
dans lequel une zone moyenne de rainure est de 0,1 à 10⁶ µm², et/ou dans lequel l'au moins une micro-caractéristique est alignée par rapport au dispositif, et/ou
dans lequel les couches polymères (13a, 13b) sont pourvues d'au moins un accès, l'au moins un accès fournissant un accès à au moins l'un parmi un plot métallique, un IC, un capteur, tel qu'un capteur optique, et un dispositif de chauffage, et/ou
un substrat rigide formant la paroi de microchambre (12), tel qu'un substrat de silicium, et/ou
dans lequel l'au moins une première microchambre (10a) a une forme ressemblant à un organisme ou tissu biologique à recevoir.

5. Dispositif microfluidique selon l'une quelconque des revendications 1 à 4, comprenant au moins une électrode (41) dans le fond (11), dans lequel l'électrode (41) est de préférence prévue dans la couche supérieure de fond (1 la),
dans lequel une électrode au niveau d'une extrémité est en contact électrique avec au moins un pilier (20), et dans lequel l'électrode comprend un contact (41a) au niveau d'une autre extrémité de celui-ci, tel qu'un plot, dans lequel éventuellement l'électrode (41) est intégrée dans un matériau isolant (41b),
et dans lequel éventuellement le contact (41a) est séparé électriquement de la paroi (12) par un autre matériau isolant (41c).

6. Dispositif microfluidique selon l'une quelconque des revendications 1 à 5, dans lequel la membrane poreuse (13) et/ou l'au moins un pilier (2) est une construction choisie parmi une construction rigide et une construction flexible.

7. Dispositif microfluidique selon l'une quelconque des revendications 1 à 6, dans lequel le fond comprend une couche métallique (13c),
de préférence entre une couche supérieure (13a) et une couche inférieure (13b), dans lequel la couche métallique est de préférence structurée, et dans lequel la couche métallique est adaptée pour détecter une déformation de l'au moins un pilier.

8. Dispositif microfluidique selon l'une quelconque des revendications 1 à 7,
dans lequel l'au moins un pilier (20) a une section transversale choisie parmi une forme carrée, rectangulaire, ovale, elliptique, circulaire, triangulaire, multigonale, et leurs combinaisons, et/ou
dans lequel une section transversale est sensiblement constante du fond (11) vers le haut, ou dans lequel la section transversale augmente progressivement en zone depuis le fond (11) vers le haut, et/ou
dans lequel au moins un pilier est pourvu d'un dispositif de guide optique (23) au niveau d'un sommet de celui-ci, tel qu'un pointeur, tel qu'un pointeur triangulaire, éventuellement dans lequel les dispositifs de guides optiques de piliers opposés peuvent pointer dans la même direction, ou dans une direction opposée, et leurs combinaisons, et/ou
dans lequel au moins un pilier (20) est creux, de préférence dans lequel la partie creuse du pilier (20) est en liaison micro-fluidique avec un premier canal (30), ou avec un second canal (31), ou avec une première ou une seconde microchambre (10a, 10b).

9. Dispositif microfluidique selon l'une quelconque des revendications 1 à 8, comprenant en outre incorporé dans le dispositif au moins l'une parmi une pompe, ou dans lequel le dispositif est adapté pour recevoir un fluide à partir d'une pompe, une soupape, une jauge de contrainte, un actionneur, un dispositif de chauffage, un refroidisseur, un capteur d'écoulement, un capteur de température, un capteur de pH, un circuit IC, un amplificateur, un actionneur, une plaque chaude, un réseau de microélectrodes, un capteur d'ions, un régulateur de pression, d'autres éléments microfluidiques, au moins l'un parmi une puce, un capteur intégré et une sortie (18), de préférence incorporé dans le fond (11), tel que dans une partie rigide de celui-ci, et/ou dans lequel une section humide/mouillée et une section sèche du dispositif sont physiquement séparées, dans lequel la section sèche comprend l'électronique.

10. Dispositif microfluidique selon l'une quelconque des revendications 1 à 9, dans lequel le fond (11) comprend un film polymère (13) qui est étirable ayant une résistance à la traction de > 1 [MPa] (norme ISO 527), et/ou souple avec un module de Young de < 3 [GPa] (norme ISO 527), ou dans lequel le film polymère est rigide ayant un module de Young de > 10 [GPa] (norme ISO 527).

11. Dispositif microfluidique selon l'une quelconque des revendications 1 à 10, comprenant en outre un support (60), tel qu'une plaque, dans lequel le dispositif microfluidique est de préférence attaché de manière détachable audit support (60).

12. Dispositif microfluidique selon l'une quelconque des revendications 1 à 11, comprenant au moins un organisme vivant ou une partie vivante de celui-ci, dans lequel l'au moins un organisme vivant est choisi parmi des cellules indifférenciées, des cellules différenciées, des cellules matures, des cellules souches, telles que des cellules primaires adhérentes ou en suspension, des cellules endothéliales, des lignées cellulaires transfectées ou non transfectées, des cellules souches pluripotentes adultes, embryonnaires ou induites, des tissus, des inserts de tissus et des micro-tissus 3D, tels que des micro-tissus cardiaques et musculaires, des cultures 3D, telles que des sphéroïdes et des organoïdes, et leurs combinaisons.

13. Procédé utilisant le dispositif selon l'une quelconque des revendications 1 à 11, comprenant l'ensemencement de la première microchambre (10a) avec des cellules musculaires squelettiques ou cardiaques de cellules souches pluripotentes ou induites, avec ou sans myoblastes/fibroblastes/cellules endothéliales pour permettre la croissance d'un faisceau musculaire ancré aux piliers ; et l'ensemencement du canal (30) avec des cellules endothéliales dérivées de cellules souches pluripotentes primaires, transfectées ou induites pour créer une culture perfusable 3D et/ou pour vasculariser le faisceau à travers la membrane poreuse.

14. Procédé d'application d'un stimulus à au moins un organisme vivant ou une partie vivante de celui-ci, comprenant
la fourniture d'au moins un dispositif microfluidique (100) selon l'une quelconque des revendications 1 à 12,
la fourniture d'au moins un organisme vivant ou d'une partie vivante de celui-ci chacun comprenant individuellement au moins une cellule,
la fourniture d'au moins un stimulus, et
l'obtention d'un résultat de test.

15. Procédé selon la revendication 14, dans lequel le stimulus est un stimulus chimique, un stimulus mécanique, un stimulus électrique, ou un stimulus optique, tel que pour un test de toxicité de médicaments et de xénobiotiques, pour un test d'efficacité, pour la modélisation des tissus barrières in vitro, pour des d'évaluation d'intégrité et des essais de transport de médicament, pour des études de métabolisme de médicament, pour des études pharmacocinétiques et toxicocinétiques de médicament, pour la métabolisation des organes et le ciblage des organes pharmacologiques, pour la modélisation de maladies, pour le diagnostic de la maladie, pour l'étude d'un mécanisme de maladie, pour le pronostic, pour la médecine personnalisée et précise, pour le dopage, pour les astronautes, pour l'interaction de médicaments, pour la maturation cellulaire, et pour la différenciation cellulaire.

16. Procédé selon l'une quelconque des revendications 14 à 15, dans lequel l'au moins un organisme vivant ou partie vivante de celui-ci est choisi parmi des cellules indifférenciées, des cellules différenciées, des cellules matures, des cellules souches, telles que des cellules primaires adhérentes ou de suspension, des cellules endothéliales, des lignées cellulaires transfectées et non transfectées, des cellules adultes, des cellules souches pluripotentes embryonnaires et induites, des tissus, des inserts de tissus, des cellules en grappe, des cellules imprimées, un organoïde, une biopsie de tissu, un tissu tumoral, un matériau de tissu réséqué, un explant d'organe, un corps embryonnaire, et des micro-tissus 3D, tels que des micro-tissus cardiaques et musculaires, des cultures 3D, telles que des sphéroïdes et des organoïdes, et leurs combinaisons.
